(19) Europäisches Patentamt
European Patent Office
Office européen des brevets

(11) **EP 4 663 625 A1**

(12) **EUROPEAN PATENT APPLICATION**

(43) Date of publication:
**17.12.2025 Bulletin 2025/51**

(21) Application number: **24181129.8**

(22) Date of filing: **10.06.2024**

(51) International Patent Classification (IPC):
*C07C 273/16* (2006.01)     *C01C 1/08* (2006.01)
*C02F 1/02* (2023.01)

(52) Cooperative Patent Classification (CPC):
**C07C 273/16; C02F 1/025;** C01C 1/086;
C02F 2101/34; C02F 2101/38; C02F 2103/005

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Yara International ASA
0277 Oslo (NO)**

(72) Inventor: **Porro, Lino Giovanni
1040 Etterbeek (BE)**

(74) Representative: **De Clercq & Partners
Edgard Gevaertdreef 10a
9830 Sint-Martens-Latem (BE)**

(54) **A DEVICE AND METHOD FOR HYDROLYZING UREA**

(57)     The present application provides for a urea hydrolyzer using only electricity as heat source for decomposing urea, thus allowing a urea plant to lower its steam requirement and its carbon dioxide emissions. The urea hydrolyzer according to the present disclosure comprises a vertical body, the vertical body comprising a top end and a bottom end, a liquid inlet for an aqueous solution comprising urea, a liquid outlet for an aqueous solution depleted in urea, and a gas outlet; an electric device located inside the body for transforming the water comprised in the aqueous solution into steam; and an electric outlet located on the vertical body, the electric outlet being electrically connected to the electric device, and configured to be connected to a power source.

The present application furthers provides a method for removing urea from an aqueous solution comprising urea, the method comprising connecting the electric outlet of a urea hydrolyzer according to the first aspect of the present disclosure to a power source; directing an aqueous solution comprising urea to the liquid inlet of the urea hydrolyzer; and recovering an aqueous solution depleted of urea from the liquid outlet of the urea hydrolyzer and a gaseous stream comprising ammonia, carbon dioxide, and water from the gas outlet.

EP 4 663 625 A1

## Description

## Field of the disclosure

The present disclosure is related to the field of urea production.

## Background information

Urea is one of the most important chemicals industrially produced today, around 200 million tons of urea are produced worldwide every year. Most of it (above 90% of total production) is used as a fertilizer in agriculture as a nitrogen source. Urea is produced by reacting ammonia ($NH_3$) and carbon dioxide ($CO_2$) in a two-step process: first, two molecules of ammonia react with one molecule of carbon dioxide to form ammonium carbamate ($H_2N$-$COONH_4$); secondly, ammonium carbamate, which will be referred to as carbamate in the remainder of this document, decomposes into urea and water. Carbon dioxide and ammonia are mixed under high pressure and high temperature in a container, called a urea reactor or urea synthesis reactor. The solution produced in the urea reactor is an aqueous solution comprising urea, ammonium carbamate, free ammonia, and water.

This solution is then processed in a number of steps to remove the ammonium carbamate and free ammonia to obtain an aqueous urea solution comprising, urea, water, and as little pollutants as possible. This aqueous urea solution is concentrated in one or more evaporators to obtain a urea melt, which comprises at least 95 weight% of urea. The urea melt can be transformed into a solid, particulate, urea-based composition, such as urea prills or granules, or into a liquid product, such as diesel-exhaust fluid, which is an aqueous solution comprising around 32.5 weight% of urea.

During the concentration of the aqueous urea solution into a urea melt, vapors comprising ammonia, carbon dioxide, urea, and water are produced and condensed to obtain an aqueous solution comprising urea, and ammonium ions, such as ammonium carbamate. It is highly desirable to remove urea, carbon dioxide, and ammonium ions from this aqueous solution in order to obtain an aqueous solution with a low level in pollutants. This aqueous solution can be re-used in the plant or safely discarded in the environment.

The removal of urea from an aqueous solution requires an input of heat to decompose the urea into ammonia and carbon dioxide, which may escape the aqueous solution as gas or may be separated from the aqueous solution downstream of the urea hydrolyzer. This is conventionally done in a device called a urea hydrolyzer using steam as heat source. However, that steam is usually produced by heating water using fossil fuels, thereby generating carbon dioxide, a greenhouse gas, which is released in the atmosphere. There is a strong desire in the fertilizer industry today to reduce the carbon footprint of its product, and reducing the carbon dioxide emissions of the production process is one way to achieve that, so there is a need to develop devices and methods for producing urea with a lower carbon footprint.

## Summary of the disclosure

A urea hydrolyzer using only electricity as heat source for decomposing urea has been designed. The urea hydrolyzer allows the plant to lower its steam requirement, therefore lowering its carbon dioxide emissions.

In a first aspect, the present disclosure provides a urea hydrolyzer for removing urea from an aqueous solution comprising urea and water, wherein the urea hydrolyzer comprises:

- a vertical body, the vertical body comprising a top end and a bottom end, a liquid inlet for an aqueous solution comprising urea, a liquid outlet for an aqueous solution depleted in urea, and a gas outlet;
- an electric device located inside the body for transforming the water comprised in the aqueous solution into steam; and
- an electric outlet located on the vertical body, the electric outlet being:

  - electrically connected to the electric device, and
  - configured to be connected to a power source.

In another aspect, the present disclosure provides a method for removing urea from an aqueous solution comprising urea, the method comprising:

  a. connecting the electric outlet of a urea hydrolyzer according to the first aspect of the present disclosure to a power source;
  b. directing an aqueous solution comprising urea to the liquid inlet of the urea hydrolyzer; and
  c. recovering an aqueous solution depleted of urea from the liquid outlet of the urea hydrolyzer and a gaseous stream comprising ammonia, carbon dioxide, and water from the gas outlet.

In another aspect, the present disclosure provides the use of a urea hydrolyzer according to the present disclosure for removing urea from an aqueous solution comprising urea.

## Brief description of the figures

The following description of the figure of a specific embodiment of a system according to the present disclosure is only given by way of example and is not intended to limit the present explanation, its application or use. In the figure, identical reference numerals refer to the same or similar parts and features.

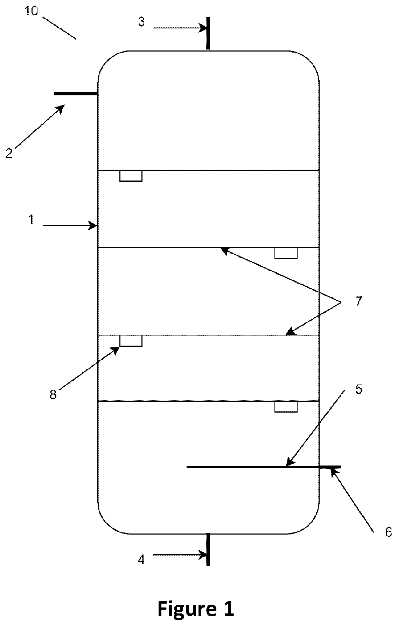

Figure 1 shows an embodiment of a urea hydrolyzer according to the present disclosure.

**Detailed description of the disclosure**

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g., component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a urea hydrolyzer for removing urea from an aqueous solution comprising urea and water, wherein the urea hydrolyzer comprises:

- a vertical body, the vertical body comprising a top end and a bottom end; a liquid inlet for an aqueous solution comprising urea, a liquid outlet for an aqueous solution depleted in urea, and a gas outlet;

- an electric device located inside the vertical body for transforming the water comprised in the aqueous solution into steam; and

- an electric outlet located on the vertical body, the electric outlet being:

    - electrically connected to the electric device, and
    - configured to be connected to a power source.

In conventional urea hydrolyzers, steam is injected at the bottom of the urea hydrolyzer and moves counter-current to the flow of the aqueous solution comprising urea. Instead of injecting steam, the urea hydrolyzer according to the present disclosure comprises an electric device, located inside its body, which is configured to transform some of the water comprised in the aqueous solution injected in the urea hydrolyzer into steam. This means that the urea plant can lower its steam production using fossil fuel sources that generate carbon dioxide emissions, and when the electricity provided to the urea hydrolyzer is created, within the urea plant or outside thereof, with non-fossil sources, such as hydraulic, wind, sea, and nuclear, the carbon footprint of the plant is greatly reduced.

For vertical urea hydrolyzers using steam as heat source, it has been shown that a counter-current setup, i.e., wherein the aqueous solution comprising urea and steam are flowing in opposite directions, enables a better urea removal.

So, the urea hydrolyzer according to the first aspect comprises a vertical body, the vertical body comprising a top end and a bottom end, a liquid inlet for an aqueous solution comprising urea, a liquid outlet for an aqueous solution depleted in urea, and a gas outlet. The urea hydrolyzer is configured such that the aqueous solution comprising urea is injected near the top end of the urea hydrolyzer via a liquid inlet, flows down the length of the urea hydrolyzer by gravity, and an aqueous solution depleted of urea is recovered at the bottom end of the urea hydrolyzer.

The vertical body has a given length, a bottom end, a top end, and may have a symmetric geometry, for example cylindrical. The length of the vertical body is defined as the distance between the top end and the bottom end.

An electric device is located inside the body for transforming a portion of the water comprised in the aqueous solution into steam. The steam created travels upwards, interacting and heating the aqueous solution as it travels, stripping part of the ammonia and carbon dioxide comprised in the aqueous solution, and exits the urea hydrolyzer via a gas outlet located at or near the top end of the urea hydrolyzer along with some ammonia and carbon dioxide. When the aqueous solution is heated above a certain temperature, the urea comprised therein decomposes into ammonia and carbon dioxide. These two elements can escape the aqueous solution into the gaseous phase and can leave the urea hydrolyzer via the gas outlet. Some ammonia and carbon dioxide may remain in the aqueous solution and can be removed from it downstream of the urea hydrolyzer. Some of the steam created by the electric device may be condensed when interacting with the aqueous solution.

In some embodiments, the urea hydrolyzer according to the first aspect comprises one or more element for increasing the contact area between the aqueous solution and steam, in particular wherein the one or more elements are perforated trays. The amount of heat transferred between a gas phase, here steam, and a liquid phase, here the aqueous solution comprising urea, is highly dependent on the contact area of the two phases. So, it may be an advantage for the urea hydrolyzer to comprises one or more element to increase said contact area. Different element designs are known in the field and can be used. A perforated tray is a possible choice of element to increase the contact area. A perforated tray can collect a certain amount of liquid phase flowing on it, and the perforations allows the gas phase to go through the tray and create bubbles that flow into the liquid phase, thereby greatly increasing the contact area between the two phases.

In some embodiments, the urea hydrolyzer according to the first aspect comprises two or more perforated trays. In some embodiments, the urea hydrolyzer according to the first aspect comprises a plurality of perforated trays.

In some embodiments, the urea hydrolyzer according to the first aspect comprises at least 5, 10, 15 or 20 perforated trays. The number of perforated trays comprised in the urea hydrolyzer may depend on the size of the urea hydrolyzer, the urea content of the aqueous solution directed to the urea hydrolyzer, and the desired urea content of the aqueous solution at the liquid outlet of the urea hydrolyzer.

In some embodiments, the one or more perforated trays have a surface ranging from 80 to 100% of the surface of the cross-section of the vertical body.

In some embodiments, the perforated trays are positioned parallel to each other and parallel to a cross-section of the vertical body. When the perforated trays are positioned parallel to each other and parallel to a cross-section of the vertical body, the flow of the liquid and gas phases are easier to predict, model, and/or control.

In some embodiments, the distance between two consecutive perforated trays is constant along the length of the urea hydrolyzer.

In some embodiments, the distance between two consecutive perforated trays is ranging from 0.5 to 2.5 meters. The distance between two consecutive perforated trays may vary depending on several factors, such as the body length of the urea hydrolyzer, the urea removal capacity of the urea hydrolyzer, and the temperature and pressure inside the urea hydrolyzer in operation.

In some embodiments, a perforated tray or the perforated trays comprise two types of through-holes: a first series of through-holes to allow the steam to move upward, and a second series of through-holes to allow the liquid to move downward. The through-holes of the first series may have a diameter smaller than the through-holes of the second series. In some embodiments, the second series of through-holes comprises one or two through-holes.

In some embodiments, the second series of through-holes in two consecutive perforated trays are at symmetrical positions related to the central axis of the urea hydrolyzer. Such a configuration increases the distance traveled by the solution, thereby increasing the interaction time between the liquid and gas phases, and the amount of heat transferred from the steam to the aqueous solution.

In some embodiments, the electric device is located closer to the bottom end than the top end, in particular wherein the distance between the electric device and the bottom end is ranging from 1 to 20% of the length of the vertical body. The closer the electric device is to the bottom end of the urea hydrolyzer, the longer the steam can interact with the aqueous solution inside the body of the urea hydrolyzer.

In some embodiments, the electric device is a resistor. A resistor is a device that has a certain resistance to the passage of electric current, and the resistor generates heat when electric current is passed through. This heat can be used to transform liquid water into steam inside the urea hydrolyzer.

In some embodiments, the electric device is configured to deliver from 0.5 to 4.0 MW of heat. The amount of heat required to be delivered by the electric device may depend on several factors, such as the flow rate of aqueous solution into the urea hydrolyzer, the urea content of the aqueous solution at the liquid inlet of the urea hydrolyzer, the expected urea content at the liquid outlet of the urea hydrolyzer.

If the electric device is a resistor, the characteristics of the resistor, for example its Ohm value can be calculated based on the expected heat (in W) to be provided by the resistor and the current voltage (in V) to be used. For example, if a resistor is expected to provide 2.1 MW of power (P) using a 4000 Volt 3-phase electric supply, the resistor should have an Ohm value (R) of 13.4 according to Equation 1.

$$R = \frac{V^2 \cdot \sqrt{3}}{P} \qquad \text{(Equation 1)}$$

In some embodiments, the urea hydrolyser is configured to receive an aqueous solution in its liquid inlet having a temperature ranging from 160 to 220°C, from 160 to 210 °C, from 170 to 220°C, from 170 to 210 °C, from 160 to 200°C, from 170 to 200 °C, or from 180 to 200°C.

In some embodiments, the urea hydrolyser is configured such that the aqueous solution recovered from its liquid outlet has a temperature ranging from 180 to 240°C, from 180 to 230 °C, from 190 to 240°C, from 190 to 230 °C, from 180 to 220°C, from 190 to 220 °C, or from 200 to 220 °C.

In some embodiments, the urea hydrolyser is configured to have an internal pressure ranging from 2.0 to 5.0 MPa, from 2.0 to 4.5 MPa, or from 2.0 to 4.0 MPa.

In some embodiments, the liquid inlet is located closer to the top end than the bottom end, in particular wherein the distance between the liquid inlet and the top end is ranging from 1 to 20% of the length of the vertical body.

In another aspect, the present disclosure provides a method for removing urea from an aqueous solution comprising urea, the method comprising:

a. connecting the electric outlet of a urea hydrolyzer according to the first aspect of the present disclosure to a power source;
b. directing an aqueous solution comprising urea to the liquid inlet of the urea hydrolyzer; and
c. recovering an aqueous solution depleted of urea from the liquid outlet of the urea hydrolyzer and a gaseous stream comprising ammonia, carbon dioxide, and water from the gas outlet.

The urea hydrolyzer requires two streams to operate: electric power to transform water into steam, and an aqueous solution comprising urea to be depleted of urea. It may be required to wait for the electric device to reach a certain temperature or a certain status for it to be able to convert water into steam before the aqueous solution is directed inside the body of the urea hydrolyzer. The steam may be created when the aqueous solution comes into contact with the electric device. The temperature of the steam created is equal to the temperature of the aqueous solution contacting the electric device.

As the aqueous solution travels down the urea hydrolyzer, it absorbs heat from the steam and the urea comprised therein decomposes into ammonia and carbon dioxide, which may escape the aqueous phase into the gaseous phase, with the help of the countercurrent flow of steam. At the bottom end of the urea hydrolyzer, an aqueous solution depleted in urea is recovered at the liquid outlet. At the top end of the urea hydrolyzer, a gaseous stream comprising water, ammonia, and carbon dioxide is recovered via the gas outlet. This stream can be sent back to another section of the urea plant, for example the synthesis section, or the recovery section, to increase the yield of the plant. Alternatively, the gaseous stream can be directed to a different production unit that requires ammonia and/or carbon dioxide.

In some embodiments, the aqueous solution directed to the liquid inlet has a urea concentration ranging from 0.2 to 2.0 weight%. The urea concentration of the aqueous solution may depend on the configuration of the plant, and the technology it uses.

In some embodiments, the aqueous solution directed to the liquid inlet has a temperature ranging from 160 to 220 °C, from 160 to 210 °C, from 170 to 220 °C, from 170 to 210 °C, from 160 to 200 °C, from 170 to 200 °C, or from 180 to 200 °C.

In some embodiments, the aqueous solution recovered from the liquid outlet has a temperature ranging from 180 to 240 °C, from 180 to 230 °C, from 190 to 240 °C, from 190 to 230 °C, from 180 to 220 °C, from 190 to 220 °C, or from 200 to 220 °C.

In some embodiments, the aqueous solution recovered from the liquid outlet has a urea concentration ranging from 0.0001 to 0.01 weight%. In some embodiments, the aqueous solution recovered from the liquid outlet has a urea concentration ranging from 100 to 1 ppm (parts per million per weight). The aqueous solution recovered at the bottom end may be used in different manners: for example, it can be added to an aqueous solution comprising urea to dilute said solution and create a product called diesel exhaust fluid, it may also be used as cooling water in the urea plant, the aqueous solution may also be sent to a boiler to produce steam. Different applications may have different purity requirements, for example water to be sent to a boiler has one of the highest purity requirement. Removing more urea of course cost more, so it may be an advantage to tune to the urea hydrolyzer to obtain exactly the urea concentration that is desired.

In some embodiments, the pressure inside the urea hydrolyzer in operation is ranging from 2.0 to 5.0 MPa, from 2.0 to 4.5 MPa, or from 2.0 to 4.0 MPa. The pressure inside the urea hydrolyzer may be controlled by a pressure valve connected to the gas outlet of the urea hydrolyzer.

In another aspect, the present disclosure provides the use of a urea hydrolyzer according to the present disclosure for removing urea from an aqueous solution comprising urea.

Figure 1 shows an embodiment of a urea hydrolyzer (10) according to the present disclosure. The urea hydrolyzer (10) comprises a vertical body(1), the vertical body (1) comprising a top end and a bottom end, a liquid inlet (2) for an aqueous solution comprising urea, a liquid outlet (4) for an aqueous solution depleted in urea, and a gas outlet (3), an electric device (5) located inside the vertical body (1) for transforming the water comprised in the aqueous solution into steam, and an electric outlet (6) located on the vertical body (1), the electric outlet being electrically connected to the electric device (5), and configured to be connected to a power source. The urea hydrolyzer (10) also comprises four perforated trays (7) for increasing the contact area between the aqueous solution and steam. Each perforated tray (7) comprises a through-hole (8) for allowing the liquid to move downward towards the bottom end of the urea hydrolyzer (10). The through-holes (8) of two consecutive perforated trays (7) are located at opposite positions related to the center of a cross-section of the vertical body (1).

**Claims**

1. A urea hydrolyzer (10) for removing urea from an aqueous solution comprising urea and water, wherein the urea hydrolyzer comprises:

   - a vertical body (1), the vertical body (1) comprising a top end and a bottom end, a liquid inlet (2) for an aqueous solution comprising urea, a liquid outlet (4) for an aqueous solution depleted in urea, and a gas outlet (3);
   - an electric device (5) located inside the vertical body (1) for transforming water comprised in the aqueous solution into steam; and
   - an electric outlet (6) located on the vertical body (1), the electric outlet (6) being:

      ◦ electrically connected to the electric device (5), and
      ◦ configured to be connected to a power source.

2. The urea hydrolyzer according to claim 1, further comprising one or more element (7) located inside the vertical body (1) for increasing the contact area between the aqueous solution and steam, in particular wherein the one or more elements are perforated trays (7).

3. The urea hydrolyzer according to claim 2, wherein the one or more perforated trays (7) have a surface ranging from 80 to 100% of the surface of the cross-section of the vertical body (1).

4. The urea hydrolyzer according to claim 2 or 3, wherein the perforated trays (7) are positioned parallel to each other and parallel to a cross-section of the vertical body (1).

5. The urea hydrolyzer according to any one of claims 2 to 4, wherein the distance between two consecutive perforated trays (7) is ranging from 0.5 to 2.5 meters.

6. The urea hydrolyzer according to any one of claims 1 to 5, wherein the electric device (5) is located closer to the bottom end than the top end, in particular wherein the distance between the electric device (7) and the bottom end is ranging from 1 to 20% of the length of the vertical body.

7. The urea hydrolyzer according to any one of claims 1 to 6, wherein the electric device is a resistor.

8. The urea hydrolyzer according to any one of claims 1 to 7, wherein the liquid inlet (2) is located closer to the top end than the bottom end, in particular wherein the distance between the liquid inlet (2) and the top end is ranging from 1 to 20% of the length of the vertical body.

9. The urea hydrolyzer according to any one of claims 1 to 8, wherein the electric device is configured to deliver from 0.5 to 4.0 MW of heat.

10. A method for removing urea from an aqueous solution comprising urea, the method comprising:

    a. connecting the electric device of a urea hydrolyzer according to any one of claims 1 to 9 to a power source;
    b. directing an aqueous solution comprising urea to the liquid inlet of the urea hydrolyzer; and
    c. recovering an aqueous solution depleted of urea from the liquid outlet of the urea hydrolyzer and a gaseous stream comprising ammonia, carbon dioxide, and water from the gas outlet.

11. The method according to claim 10, wherein the aqueous solution directed to the liquid inlet has a urea concentration ranging from 0.2 to 2.0 weight%.

12. The method according to claim 10 or 11, wherein the aqueous solution recovered from the liquid outlet has a urea concentration ranging from 0.0001 to 0.01 weight%.

13. The method according to any one of claims 10 to 12, wherein the aqueous solution directed to the liquid inlet has a temperature ranging from 160 to 220 °C.

14. Use of a urea hydrolyzer according to any one of claims 1 to 9 for removing urea from an aqueous solution comprising urea.

**Figure 1**

| | Europäisches Patentamt European Patent Office Office européen des brevets | **EUROPEAN SEARCH REPORT** | **Application Number** EP 24 18 1129 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/061780 A1 (SCHLUMBERGER TECHNOLOGY CORP [US]; SCHLUMBERGER CA LTD [CA] ET AL.) 1 April 2021 (2021-04-01) * paragraphs [0024], [0030], [0048]; figure 1 * | 1-4,6-8 | INV. C07C273/16 C01C1/08 C02F1/02 |
| X | CN 217 246 812 U (QINGZHOU LUGUANG LUBRICATING OIL CO LTD) 23 August 2022 (2022-08-23) * figures 1,2 * | 1-4,6-8 | |
| X | CN 208 626 634 U (SICHUAN TIANHUA CO LTD) 22 March 2019 (2019-03-22) * claim 1; figure 1 * | 1,6-8 | |
| X | CN 214 829 050 U (CHINA SHENHUA ENERGY CO LTD GUOHUA POWER BRANCH ET AL.) 23 November 2021 (2021-11-23) | 1-14 | |
| Y | * figure 1 * | 1-14 | |
| Y | US 7 678 267 B2 (AMI AGROLINZ MELAMINE INT GMBH [AT]) 16 March 2010 (2010-03-16) * column 2, lines 19-42; claim 6 * * column 4, lines 9-12 * | 1-14 | TECHNICAL FIELDS SEARCHED (IPC) C07C C01C C02F |
| Y | EP 2 894 130 B1 (OTKRYTOE AKTSIONERNOE OBSCHESTVO RES & DESIGN INSTITUTE OF UREA AND OR) 2 October 2019 (2019-10-02) * comparative example 2 * | 1-14 | |
| Y | CN 113 461 028 A (XIAN THERMAL POWER RES INST CO) 1 October 2021 (2021-10-01) * claim 1 * | 1-14 | |
| Y | CN 112 755 783 A (DALIAN BEST ENV EQUIPMENT CO LTD) 7 May 2021 (2021-05-07) * claim 1; figure 1 * | 1-14 | |

-/--

The present search report has been drawn up for all claims

| Place of search | Date of completion of the search | Examiner |
|---|---|---|
| The Hague | 11 November 2024 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 18 1129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KR 2022 0140664 A (KANG HAE MOON [KR]; SEO KANG YOUNG [KR]) 18 October 2022 (2022-10-18) * claim 9 * ----- | 1-14 | |
| Y | US 6 887 449 B2 (CHEMITHON CORP [US]) 3 May 2005 (2005-05-03) * figure 4 * ----- | 1-14 | |

TECHNICAL FIELDS
SEARCHED      (IPC)

The present search report has been drawn up for all claims

| Place of search | Date of completion of the search | Examiner |
|---|---|---|
| The Hague | 11 November 2024 | Matés Valdivielso, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 1129

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2024

| Patent document cited in search report | | | Publication date | Patent family member(s) | | Publication date |
|---|---|---|---|---|---|---|
| WO 2021061780 | A1 | | 01-04-2021 | NONE | | |
| CN 217246812 | U | | 23-08-2022 | NONE | | |
| CN 208626634 | U | | 22-03-2019 | NONE | | |
| CN 214829050 | U | | 23-11-2021 | NONE | | |
| US 7678267 | B2 | | 16-03-2010 | AR | 039752 A1 | 09-03-2005 |
| | | | | AT | E319658 T1 | 15-03-2006 |
| | | | | AU | 2003249863 A1 | 06-01-2004 |
| | | | | BR | 0312027 A | 22-03-2005 |
| | | | | CN | 1662452 A | 31-08-2005 |
| | | | | DE | 10229103 A1 | 15-01-2004 |
| | | | | EP | 1527019 A1 | 04-05-2005 |
| | | | | KR | 20050013622 A | 04-02-2005 |
| | | | | PL | 205698 B1 | 31-05-2010 |
| | | | | US | 2006011560 A1 | 19-01-2006 |
| | | | | US | 2009071887 A1 | 19-03-2009 |
| | | | | WO | 2004000733 A1 | 31-12-2003 |
| EP 2894130 | B1 | | 02-10-2019 | EP | 2894130 A1 | 15-07-2015 |
| | | | | RU | 2503623 C1 | 10-01-2014 |
| | | | | WO | 2014038979 A1 | 13-03-2014 |
| CN 113461028 | A | | 01-10-2021 | NONE | | |
| CN 112755783 | A | | 07-05-2021 | NONE | | |
| KR 20220140664 | A | | 18-10-2022 | NONE | | |
| US 6887449 | B2 | | 03-05-2005 | AR | 042121 A1 | 08-06-2005 |
| | | | | AU | 2003285166 A1 | 18-06-2004 |
| | | | | CA | 2502379 A1 | 10-06-2004 |
| | | | | CN | 1708456 A | 14-12-2005 |
| | | | | EP | 1562858 A1 | 17-08-2005 |
| | | | | JP | 2006507209 A | 02-03-2006 |
| | | | | TW | I338673 B | 11-03-2011 |
| | | | | US | 2004101464 A1 | 27-05-2004 |
| | | | | WO | 2004048268 A1 | 10-06-2004 |

EPO FORM P0459

For more details about this annex : see Official Journal of the European Patent Office, No. 12/82